# FASCICULE DE BREVET EUROPEEN

(11) **EP 1 745 071 B1**
(45) Date de publication et mention de la délivrance du brevet: **12.03.2014**
(21) Numéro de dépôt: 05770969.3
(22) Date de dépôt: 10.05.2005
(51) Int. Cl.: A61K 47/48, A61K 38/06, C07K 7/04, A61P 29/00, A61Q 17/04

(54) **CONJUGUES TRIPEPTIDIQUES AGONISTES DE LA MSH**
TRIPEPTIDKONJUGATE ALS MSH-AGONISTEN
MSH-AGONIST TRIPEPTIDE CONJUGATES

(30) Priorité: 11.05.2004 FR 0405069; 22.10.2004 FR 0411276
(43) Date de publication de la demande: 24.01.2007
(73) Titulaire: CENTRE NATIONAL DE LA RECHERCHE SCIENTIFIQUE (CNRS), 75016 Paris (FR); INSTITUT EUROPEEN DE BIOLOGIE CELLULAIRE, 31520 Ramonville Saint-Agne (FR); Université de Montpellier I, 34006 Montpellier Cedex 1 (FR); Université Montpellier II, 34095 Montpellier Cedex 05 (FR)
(72) Inventeur: MARTINEZ, Jean, F-34720 Caux (FR); VERDIE, Pascal, F-34270 Saint Mathieu de Treviers (FR); DUBS, Pascaline, 31100 Toulouse (FR); PINEL, Anne-Marie, F-31400 Toulouse (FR); SUBRA, Gilles, F-34990 Juvignac (FR)
(74) Mandataire: Regimbeau
(86) Numéro de dépôt international: PCT/FR2005/001165
(87) Numéro de publication internationale: WO 2005/116067

(56) Documents cités:
- WO-A-01/98362
- WO-A-95/08564
- WO-A-98/25584
- WO-A-2004/099237
- WO-A-2005/116068
- FR-A- 2 810 323
- HASKELL-LUEVANO C ET AL: "Discovery of prototype peptidomimetic agonists at the human melanocortin receptors MC1R and MC4R." JOURNAL OF MEDICINAL CHEMISTRY. 4 JUL 1997, vol. 40, no. 14, 4 juillet 1997 (1997-07-04), pages 2133-2139, XP002084110 ISSN: 0022-2623 cité dans la demande
- HASKELL-LUEVANO C ET AL: "Characterization of melanocortin NDP-MSH agonist peptide fragments at the mouse central and peripheral melanocortin receptors" JOURNAL OF MEDICINAL CHEMISTRY, AMERICAN CHEMICAL SOCIETY. WASHINGTON, US, vol. 44, no. 13, 2001, pages 2247-2252, XP002970859 ISSN: 0022-2623
- HASKELL-LUEVANO C ET AL: "Truncation studies of alpha-melanotropin peptides identify tripeptide analogues exhibiting prolonged agonist bioactivity" PEPTIDES, ELMSFORD, US, vol. 17, no. 6, 1996, pages 995-1002, XP002970858 ISSN: 0196-9781
- HRUBY V J ET AL: "ALPHA-MELANOTROPIN: THE MINIMAL ACTIVE SEQUENCE IN THE FROG SKIN BIOASSAY" JOURNAL OF MEDICINAL CHEMISTRY, AMERICAN CHEMICAL SOCIETY. WASHINGTON, US, vol. 30, no. 11, 1987, pages 2126-2130, XP001146488 ISSN: 0022-2623
- HADLEY M E ET AL: "BIOLOGICAL ACTIVITIES OF MELANOTROPIC PEPTIDE FATTY ACID CONJUGATES" PIGMENT CELL RESEARCH, MUNKSGAARD INTERNATIONAL PUBLISHERS, CAMBRIDGE, MA, DK, vol. 4, no. 4, octobre 1991 (1991-10), pages 180-185, XP008046422 ISSN: 0893-5785
- AL-OBEIDI F ET AL: "Synthesis and biological activities of fatty acid conjugates of a cyclic lactam alpha-melanotropin" JOURNAL OF MEDICINAL CHEMISTRY, AMERICAN CHEMICAL SOCIETY. WASHINGTON, US, vol. 35, 1992, pages 118-123, XP002244695 ISSN: 0022-2623
- HOLDER J R ET AL: "Characterization of aliphatic, cyclic, and aromatic N-terminally capped His-D-Phe-Arg-Trp-NH2 tetrapeptides at the melanocortin receptors" EUROPEAN JOURNAL OF PHARMACOLOGY, AMSTERDAM, NL, vol. 462, 21 février 2003 (2003-02-21), pages 41-52, XP002263329 ISSN: 0014-2999
- TODOROVIC ALEKSANDAR ET AL: "N-terminal fatty acylated His-DPhe-Arg-Trp-NH2 tetrapeptides: Influence of fatty acid chain length on potency and selectivity at the mouse melanocortin receptors and human melanocytes" JOURNAL OF MEDICINAL CHEMISTRY, vol. 48, no. 9, mai 2005 (2005-05), pages 3328-3336, XP002375935 ISSN: 0022-2623
- KOIKOV L N ET AL: "Analogs of sub-nanomolar hMC1R agonist LK-184 [Ph(CH2) 3CO-His-D-Phe-Arg-Trp-NH2]. An additional binding site within the human melanocortin receptor 1?", BIOORGANIC AND MEDICINAL CHEMISTRY LETTERS 20040802 GB LNKD- DOI:10.1016/J.BMCL.2004.05.039, vol. 14, no. 15, 2 August 2004 (2004-08-02), pages 3997-4000, ISSN: 0960-894X
- KOIKOV L.N. ET AL: "Sub-nanomolar hMC1R agonists by end-capping of the melanocortin tetrapeptide His-D-Phe-Arg-Tryp-NH2", BIOORGANIC & MEDICINAL CHEMISTRY LETTERS, vol. 13, 2003, pages 2647-2650, XP002342336,

## Description

La présente invention concerne de nouveaux conjugués tripeptidiques agonistes de la MSH et leur application thérapeutique et cosmétique.

L'alpha-MSH est une substance naturellement produite par l'organisme humain connue pour avoir de très nombreuses activités physiologiques : antipyrétiques, anti-inflammatoires et pigmentantes.

En tant que médiateur, l'alpha-MSH possède des récepteurs spécifiques dont cinq ont été décrits et possèdent chacun 7 domaines transmembranaires. Au niveau de la peau, les activités précédemment citées font intervenir le récepteur « melanocortin-1 » (MC-1r). A ce niveau, la fixation de l'alpha-MSH induit l'activation d'une protéine G (présentant une sous unité α de type Gγs) qui va elle-même stimuler l'adénylyl cyclase et ainsi produire de l'adénosine mono phosphate cyclique (ou AMPc).

La production d'AMPc induit l'activation de protéines kinases de type A qui vont activer par phosphorylation les protéines capables de se lier aux éléments de réponse à l'AMPc (ou CREB) au niveau de l'ADN des gènes de la cellule. Ceci conduit à l'expression de médiateurs qui exercent alors leurs effets sur les cellules cibles.

Chez les mammifères la coloration de la peau et des poils est due à une catégorie commune de pigments : les mélanines. La production de ces mélanines dans la peau est assurée par les mélanocytes, cellules localisées au niveau de la membrane basale de l'épiderme et dans les follicules pileux. La synthèse des mélanines est contrôlée par l'activité d'une enzyme : la tyrosinase. C'est la production de cette enzyme (ainsi que celle des enzymes associées : TRP-1 & TRP-2) qui est stimulée par la fixation de l'alpha-MSH sur son récepteur MC-1. La production de mélanine par la tyrosinase a lieu au sein d'organelles cytoplasmiques : les prémélanosomes. Une fois remplies de mélanines ces organelles sont appelées mélanosomes et sont transférées, par l'intermédiaire des dendrites du mélanocyte, aux cellules voisines : les kératinocytes. La mélanine est répartie ainsi dans l'épiderme, assurant son brunissement et sa protection.

La mélanine, pigment naturel reconnu pour ses propriétés antiradicalaires et absorbantes des rayonnements solaires, est l'agent protecteur physiologique de la peau. Il n'existe pas de composé disponible en dermocosmétologie qui puisse stimuler la production de ce pigment chez l'homme.

De plus, le mécanisme des effets anti-inflammatoires de l'alpha-MSH n'est pas totalement élucidé mais de nombreux faits expérimentaux convergent et donnent à penser que l'alpha-MSH, en se fixant sur le récepteur MC-1, inhibe l'induction de la NOSi (ou NOS2), de NFKB et induit l'expression du mRNA suivie de la production de la cytokine anti-inflammatoire IL-10. Cette cytokine s'oppose à la libération des cytokines de l'inflammation comme IL-1, IL6, IL-8, TNF-α, ou INFγ. Les kératinocytes qui constituent 95% de la population cellulaire de l'épiderme sont considérés comme des réservoirs à IL-1 et présentent à leur surface cellulaire des récepteurs MC-1. Ainsi, la fixation de l'alpha-MSH sur ces récepteurs permet une modulation des phénomènes inflammatoires locaux.

L'alpha-MSH est un tridécapeptide de formule Acétyl-Ser¹-Tyr²-Ser³-Met⁴-Glu⁵-His⁶-Phe⁷-Arg⁸-Trp⁹-Gly¹⁰-Lys¹¹-Pro¹²-Val¹³-NH₂ (ou Ser =sérine, Tyr = tyrosine, Met = méthionine, Glu = acide glutamique, His = histidine, Phe = phénylalanine, Arg = arginine, Trp = tryptophane, Gly = glycine, Lys = lysine, Pro = proline et Val = valine) De très nombreux travaux scientifiques ont établi les séquences actives de l'alpha-MSH qui sont classiquement décrites comme étant l'heptapeptide 4-10 pour les effets mélanotropes (Haskell-Luevano et al. J. Med. Chem. 1997, 40, 2133-2139) et le tripeptide 11-13 pour les effets anti-inflammatoires (Hiltz M.E. et Lipton J.M. Peptides 1990, 11, 979-982.).

De plus, les travaux récents ont conduit divers auteurs à déposer des brevets décrivant des structures cyclisées (Hruby et al. US 5 674 839 & US 6 054 556). Par ailleurs l'hexapeptide Ac-Nle-Ala-His-(D)Phe-Arg-Trp-NH₂ (FR 2 835 528) présentant la séquence tétrapeptidique de base, est commercialisé en tant qu'agoniste de l'α-MSH. Toutefois, ce composé est de haut poids moléculaire et possède donc une activité thérapeutique ou cosmétique très limitée. En effet, sa taille le rend difficile à optimiser et sa biodisponibilité est limitée. De plus, il est cher et difficile à préparer.

Le tripeptide acétylé Ac-His-DPhe-Arg-NH₂ est décrit par Haskell-Luevano et al (Peptides, 1996, Vol. 17, No. 6, pages 995-1002) comme ne présentant pas d'activité de type alpha-MSH.

Par ailleurs, le tétrapeptide Ph(CH₂)₃CO-His-DPhe-Arg-Trp possède une activité très importante en tant qu'agoniste de l'alpha-MSH (Koikov et al. Bioorganic & Medicinal Chemistry Letters, 13 (2003), pages 2647-2650). Cette séquence tétrapeptidique a toujours été identifiée comme le fragment minimal présentant une activité biologique et aucune autre modification de la taille du tétrapeptide n'a été décrite ou suggérée à ce jour. Or, la taille et le poids moléculaire de ce tétrapeptide sont encore trop importants.

De façon surprenante, les inventeurs ont découvert que certains tripeptides palmytoylés à l'extrémité N-terminale présentent encore une activité agoniste significative de l'α-MSH. Ces agonistes ont un très faible poids moléculaire, sont donc faciles à optimiser, ont une bonne biodisponibilité et sont très faciles à préparer.

La présente invention concerne donc un conjugué tripeptidique de formule :
a) A-His-DPhe-Arg-NH₂, ou
b) A-His-DPhe-Trp-NH₂
dans laquelle

A représente le radical correspondant à un acide monocarboxylique choisi parmi l'acide phénylbutyrique ou l'acide palmitique, sous forme d'énantiomères ou de diastéréoisomères ainsi que leurs mélanges, y compris les mélanges racémiques.

Les acides aminés dans le conjugué tripeptidique de formule a) ou b) peuvent avoir une configuration D, L ou DL si cela n'est pas spécifié autrement.

Ainsi, les conjugués tripeptidiques de formule a) ou b) peuvent comporter un ou plusieurs atomes de carbone asymétriques. Ils peuvent donc exister sous forme d'énantiomères ou de diastérécisomères. Ces énantiomères, diastéréoisomères, ainsi que leurs mélanges, y compris les mélanges racémiques font partie de l'invention.

Dans le cadre de la présente invention, on entend par :
- Ala, l'Alanine,
- Phe, la Phénylalanine,
- Trp, le Tryptophane,
- Arg, l'Arginine,
- Nap, la Naphtylalanine,
- Tpi, l'Acide Tétrahydronorhaman - 3 carboxylique,
- Tic, l'Acide Tétrahydroisoquinoline - 3 carboxylique,
- Orn, l'Ornithine
- His, l'Histidine et
- Lys, la Lysine.

Il est également précisé que les conjugués tripeptidiques mentionnés ci-dessus et faisant l'objet de la présente invention, sont obtenus sous la forme terminale NH₂ (autrement dit présentant une fonction amide).

Les tripeptides conjugués selon la présente invention sont liés sous forme d'amides à l'acide phénylbutyrique ou palmitique. Les conjugaisons selon la présente invention peuvent être effectués en faisant réagir la fonction amine de l'acide aminé avec la fonction acide de l'acide phénylbutyrique ou palmitique.

Parmi les conjugués tripeptidique de l'invention on peut citer les conjugués tripeptidique choisis dans le groupe constitué par :
1) Palm-His-DPhe-Arg-NH₂,
2) Palm-His-DPhe-Trp-NH₂.
3) Pbu-His-DPhe-Arg-NH₂,

Les conjugués tripeptidiques objet de la présente invention, peuvent être obtenus soit avantageusement par synthèse chimique classique, soit par synthèse enzymatique, selon des procédés quelconques connus de l'homme du métier.

La présente invention concerne en outre une composition cosmétique, dermatologique ou pharmaceutique ou un complément alimentaire comprenant un conjugué tripeptidique selon la présente invention et éventuellement un excipient cosmétiquement ou pharmaceutiquement acceptable.

Les conjugués tripeptidiques peuvent être administrés pour leur utilisation cosmétique ou pharmaceutique par voie topique. Ils peuvent aussi être utilisés dans des compléments alimentaires, autrement dit dans le domaine nutraceutique par voie orale.

Les conjugués tripeptidiques selon l'invention sont préférentiellement administrés, par voie topique.

La composition cosmétique pharmaceutique ou dermatologique pourra se présenter sous les formes qui sont habituellement connues pour ce type d'administration, c'est à dire notamment les lotions, les mousses, les gels, les dispersions, les sprays, les sérums, les masques, les laits corporels, les pommades, les solutions, les émulsions, les gels, ou les crèmes par exemple, avec des excipients permettant notamment une pénétration cutanée afin d'améliorer les propriétés et l'accessibilité du principe actif. Ces compositions contiennent généralement outre le conjugué tripeptidique selon la présente invention, un milieu physiologiquement acceptable, en général à base d'eau ou de solvant, par exemple des alcools, des éthers ou des glycols. Elles peuvent également contenir des agents tensioactifs, des conservateurs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants ou des eaux thermales.

Dans la composition cosmétique topique, le conjugué tripeptidique selon l'invention peut être présent à une concentration comprise entre 10⁻⁸ M et 10⁻³ M, avantageusement comprise entre 10⁻⁷ M et 10⁻⁵ M.

La présente invention concerne de plus un conjugué tripeptidique selon la présente invention ou une composition pharmaceutique selon la présente invention pour son utilisation en tant que de médicament, avantageusement destiné à traiter le cancer, à cicatriser les plaies et lésions chroniques des diabétiques, les ulcères variqueux ou les plaies chirurgicales, à traiter ou prévenir les vergetures, les allergies notamment cutanées, les réactions inflammatoires, les troubles de la mélanogénèse, la dermatite atopique, l'eczéma, le psoriasis, le vitiligo, l'érythème, et en particulier l'érythème photoinduit, les alopécies inflammatoires, l'asthme ou à traiter l'obésité.

Elle concerne en outre l'utilisation d'un conjugué tripeptidique selon la présente invention pour la fabrication d'un médicament destiné à traiter le cancer, à cicatriser les plaies et lésions chroniques des diabétiques, les ulcères variqueux ou les plaies chirurgicales, à traiter ou prévenir les vergetures, les allergies notamment cutanées, les réactions inflammatoires, les troubles de la mélanogénèse, la dermatite atopique, l'eczéma, le psoriasis, le vitiligo, l'érythème, et en particulier l'érythème photoinduit, les alopécies inflammatoires, l'asthme ou à traiter l'obésité.

La présente invention concerne en outre une composition cosmétique selon la présente invention pour une utilisation en tant que mélanotrope et/ou anti-érythémateux, pour accélérer la mélanisation de l'épiderme, pour obtenir un bronzage cutané naturel, pour le traitement curatif et préventif des rides du visage, du cou et des mains ou pour assurer une protection totale contre les radiations solaires ultraviolets (UVA-UVB).

Enfin, la présente invention concerne une composition cosmétique pour accélérer la mélanisation de l'épiderme, pour obtenir un bronzage cutané naturel, pour le traitement curatif et préventif des rides du visage, du cou et des mains ou pour assurer une protection totale contre les radiations solaires ultraviolets (UVA-WB) comprenant l'application sur la peau d'une composition cosmétique selon la présente invention.

Les exemples suivants sont donnés à titre indicatif non limitatif.

### Exemple 1 : Préparation de 2 tripeptides selon l'invention

Les produits chimiques utilisés sont les suivants :
Les acides aminés protégés à l'extrémité N-terminale par un groupe α-Fmoc, Fmoc-Arg(Pbf)-OH, Fmoc-D-Arg(Pbf)-OH, Fmoc-His(TrT)-OH, Fmoc-D-His(Trt)-OH, Fmoc-Phe-OH, Fmoc-D-Phe-OH, Fmoc-Trp(Boc)-OH, Fmoc-D-Trp(Boc)-OH, ont été achetés chez SENN chemicals, et chez Advanced Chemtech.

L'agent de couplage, le HBTU (hexafluorophosphate de 2-(1-H-benzotriazol-1-yl)-1,1,3,3-tétraméthyl-uronium), a été acheté chez SENN chemicals.

Le N,N-diméthylformamide (DMF), le dichlorométhane (DCM), le méthanol, l'acétonitrile, l'éther éthylique, l'acide trifluoroacétique (TFA), la pipéridine, l'anhydride acétique ont été achetés chez Riedel de Haën, Carlo Erba ou Acros organics et utilisés sans purification.

Le chlorure d'acétyle, l'anhydride acétique, la N,N-diisopropyléthylamine, le triisopropylsilane, le chlorure de benzène sulfonyle, le 3-pyridine acide propionique, l'acide butanoïque, l'acide hexanoïque, le 3-cyclohexyl-acide propionique, l'acide propionique, l'acide benzoïque, l'acide trans-cinnamique, l'acide palmitique, le chlorure de p-toluène sulfonyle, le 3,3'-diphényl-acide propionique, l'acide hexanoïque, l'acide myristique, l'acide adamantyl-carboxylique, l'acide indole-3-acétique, l'acide (2-pyrid-3-yl)thiazole-4-carboxylique, l'acide 3-thiophénacétique, l'acide 2-thiophénacétique, l'acide 2-benzimidazole propionique, l'acide 4-biphénylacétique, l'acide 4-pyridylacétique, l'acide α-méthyl-p-(2-thiénoyl)phénylacétique (suprofène) et l'acide α-méthyl-4-(2-méthylpropyl)-benzène acétique (ibuprofène) ont été achetés chez Aldrich ou Avocado. Tous les réactifs et produits chimiques étaient de qualité ACS et ils ont été utilisés sans autre purification.

Les Lanternes Polyamide Rink amide Synphase de la série D et les Lanternes Polystyrene hydroxy trityl Synphase de la série D ont été fournies par Mimotopes, Pty.

La [¹²⁵I]-NDP-MSH utilisée pour des tests de liaison par compétition a été préparée selon une procédure décrite dans l'article de Tatro, et al. (Endocrinology 1987, 121, 1900).

La synthèse peptidique en phase solide a été réalisée en utilisant la stratégie Fmoc sur un synthétiseur automatisé ACT496 Ω. Chaque puits a été rempli avec 150 mg de résine PS-Rink amide. La résine a été laissée gonfler pendant 30 minutes dans du DCM. Les étapes de déprotection et de couplage ont été réalisées jusqu'à ce que les séquences souhaitées aient été synthétisées. Pour l'étape de couplage, trois solutions ont été successivement ajoutées aux cuvettes réactionnelles : 400 µl de solution 0,5 M d'acide aminé protégé à l'extrémité N-terminale par un groupe Fmoc dans de la N-méthylpyrrolidone ; 400 µl de solution 0,5 M de N-méthylmorpholine dans du DMF et 400 µl de solution 0,5 M de HBTU dans du DMF. Le temps de couplage a été de 90 min. L'étape de déprotection de 20 min a été réalisée en utilisant 1,2 ml de DMF/pipéridine (solution 80/20, v/v). Cinq lavages de 1,5 ml ont été utilisés entre les étapes y compris 2 fois avec du DMF, 1 fois avec du DCM, 1 fois avec du méthanol et 1 fois avec du DMF.

La déprotection de la chaîne latérale et le clivage des peptides ont été réalisés pendant 2 heures dans le bloc de 96 réacteurs de l'automate en utilisant 1,5 ml de cocktail de clivage (acide trifluoroacétique/eau/triisopropylsilane, 95/2,5/2,5, v/v/v) par puits. Après l'élimination de la résine par filtration, le cocktail de clivage a été concentré dans une centrifugeuse sous vide Jouan RC10-10. Les peptides ont été précipités dans de l'éther diéthylique, transformés en culot par centrifugation, l'éther à été éliminé. Cette opération de décantation a été répétée avec de l'éther diéthylique frais. Le peptide brut a été solubilisé dans de l'acétonitrile/eau (50/50, v/v) contenant 0,1 % de TFA. Les échantillons ont été ensuite congelés à -80°C et lyophilisés. Cette opération a été répétée deux fois.

Un échantillon de 50 mg de peptide brut a été purifié par chromatographie liquide en phase inverse en utilisant le système Waters Deltaprep avec un détecteur de longueur d'onde double (214 et 254 nm) et une cartouche deltapack C₁₈ en utilisant le même système de solvant que pour l'analyse.

Les peptides simples ont été analysés par CLHP en phase inverse et CL/SM. 500 µl d'acétonitrile/eau (50/50, v/v) contenant 0,1 % de TFA ont été distribués sur les composés lyophilisés. 10 µl de chaque tube ont été prélevés pour l'analyse de CLHP et de CL/SM ESI+.

Les analyses de CLHP ont été réalisées sur un système de CLHP Waters Alliance 2690 et un détecteur à barrette de photodiodes Waters 996 et une colonne C18 Merck Chromolith Speed ROD de 50 x 4,6 mm. Un débit de 5 ml/min et un gradient de 100 % de B à 100 % de C sur 3 min ont été utilisés (Eluant B, eau/0,1 % de TFA ; Eluant C, acétonitrile/0,1 % de TFA). Les estimations de la pureté sont basées sur le pourcentage de surface des pics détectés à 214 nm.

Le système de CL/SM était constitué d'une CLHP Waters Alliance 2690 couplée à un spectromètre Micromass Platform II (mode ionisation par électronébulisation, ESI+). Toutes les analyses ont été réalisées en utilisant une colonne Waters Symmetry C18, 3,5 µm, 2,1 x 30 mm. Un débit de 600 µl/min et un gradient de 100 % de B à 100 % de C sur 3 min ont été utilisés (Eluant B, eau/0,1 % de TFA ; Eluant C, acétonitrile/0,1 % de TFA).

Les spectres de masse par électronébulisation ionique positive ont été acquis à un débit de solvant de 100 ml/min. De l'azote a été utilisé à la fois pour le gaz nébulisant et pour le gaz séchant. Les données ont été acquises en mode lecture de m/z 400 à 1400 dans des intervalles de 0,1^{-s} ; 10 lectures ont été additionnées pour produire le spectre final.

Les poids moléculaires de tous les composés ont été calculés en utilisant des masses mono-isotopiques (C = 12,000, H = 1,007, N = 14,003, O = 15,994, S = 31,972).

Les résultats pour les deux tripeptides synthétisés sont rassemblés dans le tableau 1 suivant :

**Tableau 1 : Résultats analytiques de la synthèse des tripeptides**

| **No de conjugué peptidique selon l'invention** | **Séquence** | | | | | **%** | **Poids** |
|---|---|---|---|---|---|---|---|
| | **A** | **AA1** | **AA2** | **AA3** | | **pureté** | **Moléculaire** |
| 1 | Palm | His | DPhe | Arg | NH₂ | 92 | 695,4 |
| 2 | Palm | His | DPhe | Trp | NH₂ | 97 | 725,4 |

### Exemple 2 : Propriétés biologiques des 2 conjugués tripeptides selon la présente invention

La lignée cellulaire humaine M4Be (Jacubovichet al. Cancer Immunol. Immunother. 1979, 7, 59-64), une lignée cellulaire de mélanocytes capable de produire des mélanines, a été utilisée dans cette étude pour déterminer les valeurs de CE₅₀.

Les cellules ont été maintenues dans du milieu Eagle modifié de Dulbecco avec 10 % de FCS, 1 mM de glutamine, 100 U/ml de pénicilline et 10⁻⁴ g/ml de streptomycine. Toutes les lignées cellulaires ont été maintenues à 37°C dans une atmosphère à 5 % de CO₂ et les milieux de culture des cellules ont été renouvelés tous les deux jours. Les cellules ont été plaquées sur une plaque à 96 puits (Nunc, Roskilde, Danemark) 24 heures avant le contact des peptides.

La lignée cellulaire de mélanome de souris Cloudman S91 (Yasumura et al. Science. 154, pages 1186-1189) a été utilisée pour le criblage préliminaire. Les cellules ont été maintenues dans du HAM F10 avec 10 % de sérum de cheval, 2 % de sérum de veau foetal (FCS), 1 mM de glutamine, 100 U/ml de pénicilline et 10⁻⁴ g/ml de streptomycine (Eurobio, Les Ulis, France). Toutes les lignées cellulaires ont été maintenues à 37°C dans une atmosphère à 5 % de CO₂ et les milieux de culture des cellules ont été renouvelés tous les deux jours. Les cellules ont été plaquées sur une plaque à 96 puits (Nunc, Roskilde, Danemark) 24 heures avant le contact des peptides.

### Mesure de l'AMPc :

Brièvement, les cellules plaquées la veille ont été incubées 1 heure avec 10⁻⁴ M d'adénine (Sigma) et ensuite 10 minutes dans du milieu de Krebs Ringer Hepes avec 10⁻⁴ M d'isobutyl-1-méthylxanthine (Sigma), 10⁻⁴ M de 4-[(3-butoxy-4-méthoxyphényl)méthyl]-2-imidazolidinone (Calbiochem) et des tripeptides à 10⁷ M selon l'invention.

Après ce temps, la lyse des cellules a été réalisée selon le protocole du fabricant.

La teneur en AMPc a été mesurée en utilisant un coffret de test de liaison par compétition (RPN225, Amersham Pharmacia Biotech) selon les instructions du fabricant. Chaque expérience indépendante a été réalisée au moins deux fois en triple.

Ainsi 1 tripeptide selon l'invention a été testé à une concentration de 50 nM sur l'induction d'AMPc qui a été calculée sur la réponse de la production d'AMPc induite par l'α-MSH (50 nM). Les résultats sont présentés dans le Tableau 2 suivant.

**Tableau 2 : Induction d'AMPc des tripeptides selon l'invention (donnée en pourcentage de la réponse maximale de l'hexapeptide Ac-Nle-Ala-His-(D)Phe-Arg-Trp-NH₂ à 50 nM) sur une lignée cellulaire de mélanome de souris Cloudman S91**

| **No de conjugué peptidique selon l'invention** | **Structure** | **% d'induction d'AMPc** |
|---|---|---|
| 1 | Palm-His-(D)Phe-Arg-NH₂ | 147 |

### Détermination de la CE₅₀ :

Les courbes ont été ajustées et les valeurs de CE₅₀ ont été déterminées avec la régression non linéaire dans le GraphPad Prism (logiciel GraphPad). Chaque valeur de CE₅₀ était la moyenne d'au moins deux jeux d'expériences indépendantes.

### Etudes de liaison détermination de la CI₅₀

L'affinité de liaison de l'αMSH pour le MC₁-R a été déterminée par des études de compétition avec la [¹²⁵I]-NDP-MSH. Des cellules Cos-1 transfectées ont été incubées dans du milieu Eagle modifié de Dulbecco, 0,2 % de BSA, 0,3 mM de tampon phénatroline pendant 24 heures. 10⁵ cellules ont été plaquées sur des plaques à 24 puits avec un ligand radio-marqué à une concentration de 5,8 10⁻¹¹ M. Après une incubation de 2 heures à 24°C avec diverses concentrations de ligand, des lavages sont réalisés et la radioactivité est mesurée.

Les CE₅₀ pour 2 tripeptides selon l'invention ont été rassemblés dans le tableau 3 suivant et comparés aux CE₅₀ de l'hexapeptide et de l'α-MSH

**Tableau 3 : CE₅₀ de deux tripeptides selon l'invention comparés à l'α-MSH sur la production d'AMPc dans des cellules M4Be**

| **Composés** | **Structure** | **Temps de rétention (min)** | **CE₅₀ (nM)** |
|---|---|---|---|
| α-MSH | Ac-Ser-Tyr-Ser-Met-Glu- His-Phe-Arg-Trp-Gly-Lys- Pro-Val-NH2 | 1,09 | 8,10 |
| *Hexapeptide* | Ac-Nle-Ala-His-(D)Phe- Arg-Trp-NH2 | 1,14 | 4,00 |
| 1 | Palm-His-(D)Phe-Arg-NH₂ | 1,66 | 2,3 |
| 2 | Palm-His-(D)Phe-Trp-NH₂ | 2,07 | 17,3 |

Les résultats sont en conformité avec ceux obtenus avec le Palm-tripeptides 1 [Palm-His-(D)Phe-Arg-NH₂] indiquant 147 % d'induction d'AMPc.

Le résultat le plus important et le plus frappant est l'activité des tripeptides palmitoylés au niveau du récepteur MC₁. Dans cette étude, nous avons montré que la palmitoylation des tripeptides C-terminaux de la séquence de l'α-MSH a conduit à des composés actifs au niveau du récepteur MC₁ en induisant la production d'AMPc. Ces molécules ne possèdent qu'une partie de ce qui est décrit dans la littérature comme la séquence minimale de la séquence de l'agoniste d'α-MSH.

De manière intéressante, l'élimination du résidu Tryptophane a donné naissance au tripeptide 1 qui a montré une activité dans la plage nanomolaire (CE₅₀ = 2,3 nM). Le tripeptide 2 manquant de Arg a présenté une puissance moindre, avec un CE₅₀ de 17,3 nM. Ces tripeptides sont de bonnes molécules chefs de file pour concevoir des ligands puissants actifs de l'α-MSH pour MC₁R. Ces résultats indiquent également que la présence d'une chaîne hydrophobe sur l'extrémité N-terminale des analogues de l'α-MSH, particulièrement d'un groupe palmitoyle, est un élément structural important pour l'activation du récepteur MC₁ de l'α-MSH.

## Revendications

1. Conjugué tripeptidique de formule :
a) A-His-DPhe-Arg-NH₂, ou
b) A-His-DPhe-Trp-NH₂,
dans laquelle
A représente le radical correspondant à un acide monocarboxylique choisi parmi l'acide phénylbutyrique ou l'acide palmitique, sous forme d'énantiomères ou de diastéréoisomères ainsi que leurs mélanges, y compris les mélanges racémiques.

2. Composition cosmétique, dermatologique ou pharmaceutique **caractérisée en ce qu'**elle contient un conjugué tripeptidique selon la revendication 1, et éventuellement un excipient cosmétiquement ou pharmaceutiquement acceptable.

3. Conjugué tripeptidique selon la revendication 1, ou composition pharmaceutique selon la revendication 2 à titre de médicament, avantageusement destiné à traiter le cancer, à cicatriser les plaies et lésions chroniques des diabétiques, les ulcères variqueux, les plaies chirurgicales, à traiter ou prévenir les vergetures, les allergies notamment cutanées, les réactions inflammatoires, les troubles de la mélanogénèse, la dermatite atopique, l'eczéma, le psoriasis, le vitiligo, l'érythème, et en particulier l'érythème photoincuit, les alopécies inflammatoires, l'asthme ou à traiter l'obésité.

4. Composition dermo-cosmétique selon la revendication 2 pour son utilisation en tant que mélanotrope et/ou anti-érythémateux, pour accélérer la mélanisation de l'épiderme, pour obtenir un bronzage cutané naturel, pour le traitement curatif et préventif des rides du visage, du cou et des mains ou pour assurer une protection totale contre les radiations solaires ultraviolets (UVA-UVB).

## Patentansprüche

1. Tripeptid-Konjugat der Formel:
a) A-His-DPhe-Arg-NH₂ oder
b) A-His-DPhe-Trp-NH₂,
wobei
A das Radikal darstellt, das einer Monocarbonsäure entspricht, die ausgewählt ist aus Phenylbuttersäure oder Palmitinsäure, in Form von Enantiomeren oder von Diastereoisomeren sowie deren Gemische,
einschließlich der racemischen Gemische.

2. Kosmetische, dermatologische oder pharmazeutische Zusammensetzung, **dadurch gekennzeichnet, dass** sie ein Tripeptid-Konjugat nach Anspruch 1 und gegebenenfalls einen kosmetisch oder pharmazeutisch verträglichen Träger enthält.

3. Tripeptid-Konjugat nach Anspruch 1 oder pharmazeutische Zusammensetzung nach Anspruch 2 als Medikament, vorteilhafterweise bestimmt für die Behandlung von Krebs, für die Heilung von chronischen Wunden und Verletzungen von Diabetikern, offenen Beinen, chirurgischen Wunden, für die Behandlung oder Vorbeugung von Dehnungsstreifen, Allergien, insbesondere der Haut, Entzündungsreaktionen, Beschwerden der Melanogenese, atopischer Dermatitis, Ekzemen, Psoriasis, Vitiligo, Erythema und insbesondere lichtbedingter Ehrythema, entzündlichen Alopezien, Asthma oder für die Behandlung von Fettleibigkeit.

4. Dermokosmetische Zusammensetzung nach Anspruch 2 für ihre Verwendung als melanotropische und/oder anti-erythematische Zusammensetzung, um die Melanisierung der Epidermis zu beschleunigen, um eine natürliche Hautbräunung zu erhalten, für die kurative oder präventive Behandlung von Falten im Gesicht, am Hals und an den Händen oder um einen vollständigen Schutz gegen ultraviolette Sonnenstrahlen (UVA-UVB) sicherzustellen.

## Claims

1. A tripeptide conjugate of formula:
a) A-His-DPhe-Arg-NH₂, or
b) A-His-DPhe-Trp-NH₂,
in which
A represents the radical corresponding to a monocarboxylic acid chosen from phenylbutyric acid or palmitic acid, in the form of enantiomers or of diastereoisomers, and also mixtures thereof, including racemic mixtures.

2. A cosmetic, dermatological or pharmaceutical composition, **characterized in that** it contains a tripeptide conjugate according to claim 1 and, optionally, a cosmetically or pharmaceutically acceptable excipient.

3. The tripeptide conjugate according to claim 1 or the pharmaceutical composition according to claim 2, as a medicament, advantageously for use in treating cancer, in healing chronic wounds and lesions in diabetics, varicose ulcers or surgical wounds, in treating or preventing stretchmarks, allergies, in particular skin allergies, inflammatory reactions, melanogenesis disorders, atopic dermatitis, eczema, psoriasis, vitiligo, erythema, and in particular photo-induced erythema, inflammatory alopecia or asthma, or in treating obesity.

4. The dermo-cosmetic composition according to claim 2, for use as a melanotropic and/or anti-erythematous agent, for accelerating epidermal melanization, for obtaining natural skin tanning, for the curative and preventive treatment of wrinkles on the face, neck and hands, or for providing complete protection against ultraviolet (UVA-UVB) solar radiation.
